# EUROPEAN PATENT SPECIFICATION

(11) **EP 0 875 514 B1**
(45) Date of publication and mention of the grant of the patent: **25.06.2003**
(21) Application number: 98107843.9
(22) Date of filing: 29.04.1998
(51) Int. Cl.: C07F 9/655, A61K 7/48

(54) **A process for producing an ascorbic acid derivative**
Verfahren zur Herstellung von Ascorbinsäurederivaten
Procédé de préparation de dérivés d'acide ascorbique

(30) Priority: 30.04.1997 JP 11309297
(43) Date of publication of application: 04.11.1998
(73) Proprietor: SHOWA DENKO KABUSHIKI KAISHA, Minato-ku, Tokyo (JP)
(72) Inventor: Suzuki, Masahiro, Sanbu-gun, Chiba, 299-3234 (JP); Tsuzuki, Toshi, c/o Showa Denko K.K., Chiba-shi, Chiba 267-0056 (JP); Itoh, Shinobu, c/o Showa Denko K.K., Tokyo 105-8518 (JP); Ogata, Eiji, c/o Showa Denko K.K., Tokyo 105-8518 (JP)
(74) Representative: Strehl Schübel-Hopf & Partner

(56) References cited:
- EP-A- 0 339 486
- EP-A- 0 436 936
- CHEMICAL ABSTRACTS, vol. 105, no. 24, 15 December 1986 Columbus, Ohio, US; abstract no. 213939, MOTOI T: "Skin cosmetics containing ascorbic acid derivatives" XP002073274 & JP 61 152 613 A (KANEBO, LTD.;JAPAN) 11 July 1986
- CHEMICAL ABSTRACTS, vol. 100, no. 21, 21 May 1984 Columbus, Ohio, US; abstract no. 175214, "Ascorbic acid derivatives" XP002073275 & JP 58 222 078 A (SANTEN PHARMACEUTICAL CO.) 23 December 1983

## Description

### FIELD OF THE INVENTION

The present invention relates to a novel process for producing an ascorbic acid derivative.

### BACKGROUND OF THE INVENTION

The effects of ascorbic acid include the inhibition of lipid peroxidation, acceleration of collagen formation, retardation of melanin formation, enhancement of immune functions and the like. For these purposes, ascorbic acid has hitherto been used in the fields of medical preparations, agricultural chemicals, animal drugs, foods, feeds, cosmetic preparations and the like. However, ascorbic acid has poor aging stability and poor liposolubility. Accordingly, the cumulative amount thereof in cells after permeating through the cell membrane is limited, and the physiological actions of vitamin C cannot be achieved to a satisfactory extent.

To cope with this, various derivatives have been proposed, where the hydroxyl group present in the enediol part at the 2-or 3-position, which is easily oxidized, is transformed into a phosphoric acid ester (as described, for example, in JP-B-52-1819 (the term "JP-B" as used herein means an "examined Japanese patent publication") and JP-A-02-279690 or US-A-5 110 951 (the term "JP-A" as used herein means an "unexamined published Japanese patent application")) so as to improve stability, or is acylated with a fatty acid to thereby improve liposolubility (as described, for example, in JP-A-59-170085 or EP-A-0 121 758).

Of the conventional ascorbic acid derivatives, compounds having satisfactorily improved stability (for example, magnesium L-ascorbic acid 2-phosphate) still lack adequate liposolubility.

JP-A-58-222078 proposes a 6-O-alkanoyl-ascorbic acid-2- or -3-phosphoric acid ester as a novel compound having increased stability and an appropriate solubility in lipoid. The alkanoyl group thereof has less than eleven carbon atoms, and only a pivaloyl group is disclosed as an example thereof. The compound of the present invention cannot be obtained by the production process disclosed in JP-A-58-222078. Furthermore, the novel compound disclosed therein does not have both sufficiently improved stability and liposolubility.

JP-A-61-152613 describes a cosmetic material containing a 6-O-higher acylascorbic acid-2-phosphoric acid ester. In this patent publication: first, transformation into a sulfuric acid ester but not into a phosphoric acid ester is described; second, the ascorbic acid derivative thus obtained is not identified; and third, the results of the working examples thereof are closely similar to those of the working examples of JP-A-61-151107 where the same experiment is performed except for using a 6-O-higher acylascorbic acid-2-sulfuric acid ester.

EP 0339486 and German Patent Publication DE4000397A1 propose compounds such as 6-O-octadecanoyl-2-(O*O*-diethylphosphoryl)-ascorbic acid.

### SUMMARY OF THE INVENTION

It is an object of the present invention to provide an industrial method for easily producing an ascorbic acid derivative.

Under these circumstances, the present inventors have conducted extensive investigations.

Namely, the above objectives of the present invention have been achieved by providing:
1. A process for producing an ascorbic acid derivative which is a compound represented by the following formula (1) or a salt thereof: 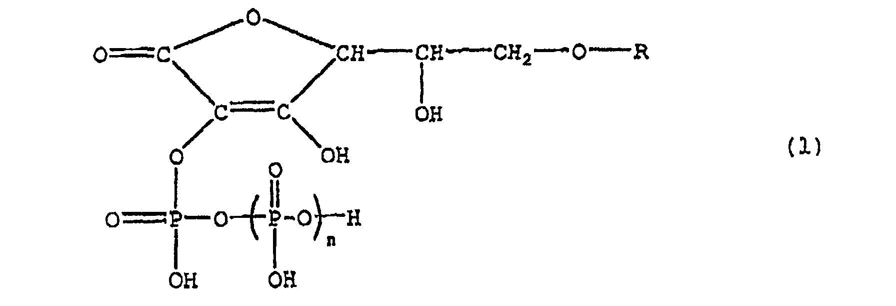 wherein R represents an acyl group having eleven or more carbon atoms and n is 0, 1 or 2, which comprises reacting an ascorbic acid-2-phosphoric acid ester or 2-pyrophosphoric acid ester or 2-triphosphoric acid ester and/or a salt thereof with at least one of a fatty acid, a fatty acid ester thereof and a salt thereof to produce a compound represented by formula (1) or a salt thereof, wherein R represents an acyl group having eleven or more carbon atoms and n is 0, 1 or 2.
2. A process for producing an ascorbic acid derivative which is a compound represented by the following formula (2) or a salt thereof, which comprises reacting an ascorbic acid-2-phosphoric acid ester and/or a salt thereof with at least one of a fatty acid, a fatty acid ester thereof and a salt thereof to produce a compound represented by the following formula (2) or a salt thereof: 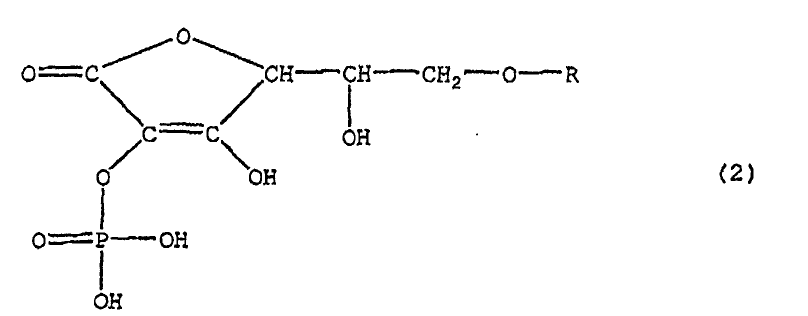 wherein R represents an acyl group having eleven or more carbon atoms.
3. The process for producing an ascorbic acid derivative as described in 1 or 2 above, wherein said reacting step comprises reacting in the presence of a condensing agent.
4. The process for producing an ascorbic acid derivative as described in 1 or 2 above, wherein said reacting step comprises reacting in concentrated sulfuric acid.

### DESCRIPTION OF THE PREFERRED EMBODIMENTS

The present invention is described in detail below.

The ascorbic acid derivative of the present invention is a compound represented by the following formula (1) or a salt thereof: 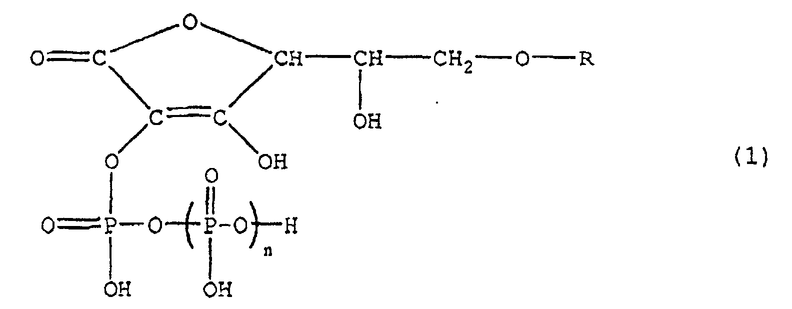 wherein R represents an acyl group and n is 0, 1 or 2. This compound is stable and difficultly oxidized because the 2-position is esterified. Furthermore, this is a monoester of a higher fatty acid having 11 or more carbon atoms, preferably from 12 to 28 carbon atoms. Therefore, the compound can have appropriate liposolubility and facilitated cellular uptake. Furthermore, because the phosphoric acid group at the 2-position is readily hydrolyzed by phosphatase in vivo, and because the higher fatty acid ester is an ester with a primary alcohol (6-position) susceptible to the action of lipase or esterase, the compound of the present invention is easily biotransformed into ascorbic acid.

The compound represented by formula (1) or a salt thereof of the present invention can be produced according to the following reaction scheme (in the case of n=0 in formula (1)) : 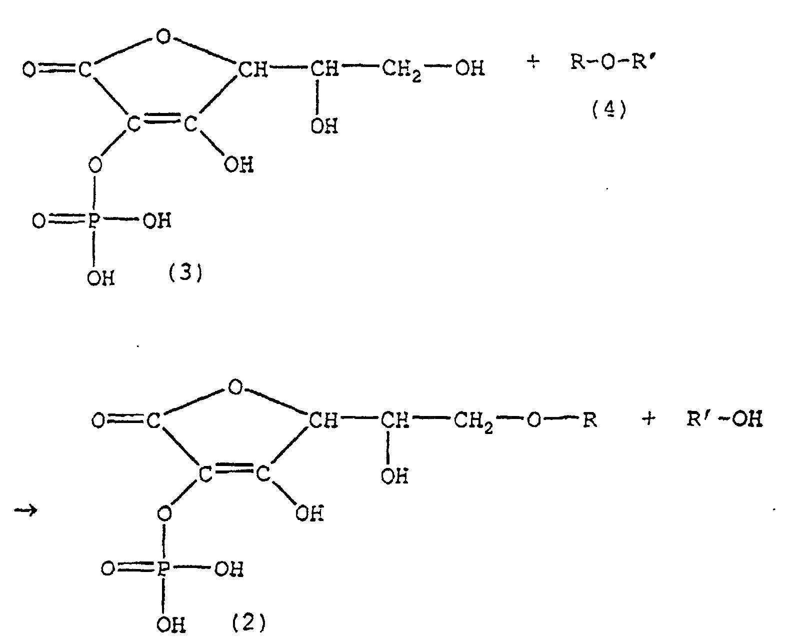 wherein R represents an acyl group having eleven carbon atoms or more and R' represents hydrogen, a cation or an alkyl group.

More specifically, an ascorbic acid-2-phosphate (3) and/or a salt thereof is reacted with (4) which is at least one of a fatty acid, an ester thereof and a salt thereof to produce an ascorbic acid-2-phosphate-6-fatty acid ester (2) or a salt thereof. This reaction is preferably conducted in the presence of a condensing agent. For example, when sulfuric acid is used as the condensing agent, concentrated sulfuric acid, an ascorbic acid-2-phosphate and a fatty acid or an ester or salt thereof are mixed and reacted. The fatty acid ester (wherein in formula (4), R' is an alkyl group) is preferably a lower alkyl ester such as a methyl ester or an ethyl ester.

The starting materials may be used in equimolar amounts. However, as long as no problems occur during purification or isolation, one part may be present in slight excess.

The reaction time and the reaction temperature vary depending on whether the fatty acid is a free acid, an ester or a salt, or the kind and the amount of the condensing agent. However, the reaction time is generally from 1 to 120 hours, preferably from 10 to 60 hours, and the reaction temperature is generally from 5 to 70°C, preferably from 15 to 30°C. The amount of water carried over from the starting materials or catalyst into the reaction solution is suitably 10% or less, preferably 2% or less.

Where a solvent is used in this reaction, the sulfuric acid as the condensing agent may be used concurrently as the solvent, or the solvent may be selected from other solvents which can dissolve the starting materials.

The purification or isolation may be performed using a known method such as solvent extraction, washing, salting out or column chromatography. For example, the ester or salt thus obtained may be isolated or purified by ether extraction or hexane washing. If desired, the ester or salt thus obtained may further be isolated or purified by reverse phase chromatography or the like.

The salt of the compound represented by formula (2) can be obtained as a salt with the corresponding base by neutralizing the ascorbic acid-2-phosphate-6-higher fatty acid ester thus obtained with an appropriate base (e.g., sodium hydroxide, potassium hydroxide, magnesium hydroxide, calcium hydroxide, ammonia, monoethanolamine, diethanolamine, triethanolamine, dicyclohexylamine), for example, in a solvent capable of dissolution, such as water or methanol.

Preferred examples of the salt include alkali metals, alkaline earth metals, aluminum, iron, zinc and bismuth. Of these, alkali metals such as sodium and potassium, and alkaline earth metals such as calcium and magnesium are more preferred.

In the compound represented by formula (2), the hydroxyl group at the 3- or 4-position may be protected by a conventionally known group which can be easily transformed to a hydroxyl group, and the present invention includes compounds having such a protective group.

This reaction can be applied not only to the production of the 6-O-higher fatty acid ester of an ascorbic acid-2-phosphate of the present invention, but also to the production of 6-O-lower fatty acid esters thereof.

Of the ascorbic acid derivatives of the present invention, the L-form is preferred in view of its vitamin C activity. Particularly, in view of liposolubility, the higher fatty acid ester at the 6-position is preferably a lauric acid ester, a myristic acid ester, a palmitic acid ester or a stearic acid ester.

### EXAMPLES

The present invention is described below by reference to the following Examples, however, the present invention should not be construed as being limited thereto.

### Example 1

### L-Ascorbic acid-2-phosphate-6-palmitate:

10 mmol (3.8 g) of magnesium L-ascorbic acid-2-phosphate was dissolved in 60 ml of concentrated sulfuric acid and to the resulting solution, 15 mmol (3.8 g) of palmitic acid was added. The mixed solution thus obtained was homogeneously stirred and after standing at room temperature for 24 hours, the reaction mixture was poured into about 300 ml of ice water. The precipitate was extracted twice with 200 ml of diethyl ether. The extracts were combined and washed with 300 ml of 2N hydrochloric acid containing 30% isopropanol, and the diethyl ether was removed by distillation under reduced pressure. The deposit was washed twice with about 200 ml of n-hexane and then dried under reduced pressure to obtain 3.2 g of L-ascorbic acid-2-phosphate-6-palmitate (yield: 65%). Various analytic data of the compound thus prepared are shown below.
MS m/z = 493 [M-H], 495 [M+H], 517 [M+Na]
¹H-NMR (400 MHz, CD₃OD) δ: 0.90 (3H, t, J=6.9Hz, 7-H), 1.29 (24H, s, 6-H), 1.63 (2H, hep, J=7.3Hz, 5-H), 2.38 (2H, t, J=7.4Hz, 4-H), 4.12-4.30 (3H, m, 2, 3-H), 4.87 (1H, t, J=1.7 Hz, 1-H)

The assignment numbers of the ¹H-NMR peaks are as follows. 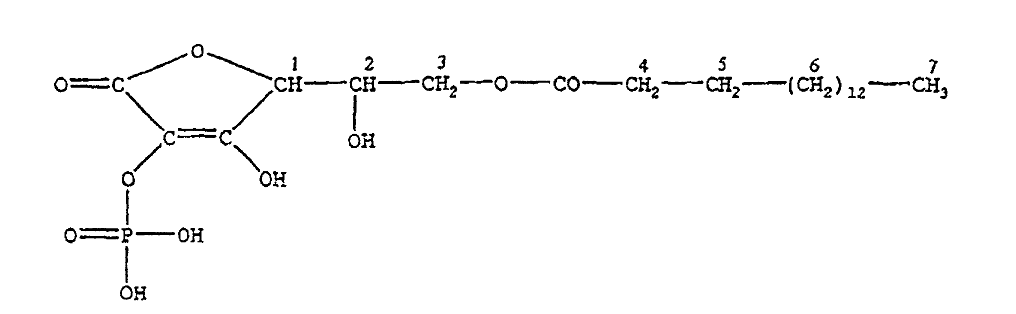 ¹³C-NMR (100 MHz, CD₃OD) δ; 14.4 (1C, s, 13-C), 23.7 (1C, 12-C), 25.9 (1C, s, 11-C), 30.2-30.7 (10C, s, 10-C), 33.0 (1C, s, 9-C), 34.9 (1C, s, 8-C), 65.5 (1C, s, 7-C), 67.9 (1C, s, 6-C), 77.2 (1C, s, 5-C), 115.3 (1C, d, J=5.7 Hz, 4-C), 160.4 (1C, d, J=3.8 Hz, 3-C), 170.5 (1C, d, J=6.1 Hz, 2-C), 175.1 (1C, s, 1-C)

The assignment number of the ¹³C-NMR peaks are as follows.

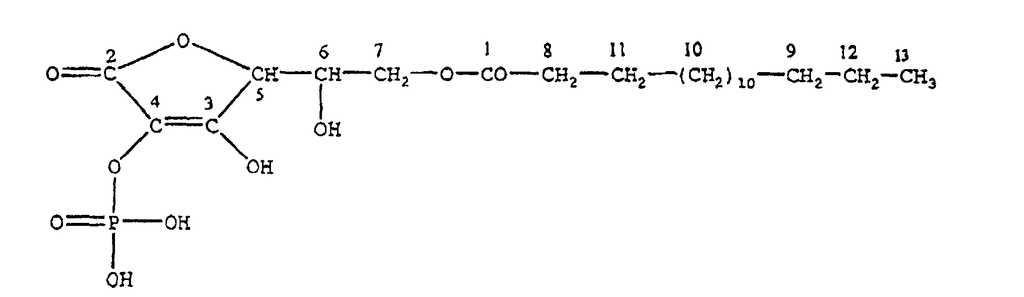

### Example 2

### L-Ascorbic acid-2-phosphate-6-palmitate:

The reaction was performed in the same manner as in Example 1, except for using sodium L-ascorbic acid 2-phosphate in place of magnesium L-ascorbic acid 2-phosphate. As a result, 3.7 g of L-ascorbic acid-2-phosphate-6-palmitate was obtained (yield: 75%).

### Example 3

### L-Ascorbic acid-2-phosphate-6-stearate:

The reaction was performed in the same manner as in Example 1, except for using methyl stearate in place of palmitic acid to obtain 4.2 g of L-ascorbic acid-2-phosphate-6-stearate (yield: 81%). Various analytic data of the compound thus obtained are shown below.
MS m/z = 521 [M-H], 523 [M+H], 545 [M+Na]
¹H-NMR (400 MHz, CD₃OD) δ: 0.90 (3H, t, J=6.8 Hz, 7-H), 1.29 (28H, s, 6-H), 1.61 (2H, hep, J=7.2 Hz, 5-H), 2.37 (2H, t, J=7.3 Hz, 4-H), 4.11 - 4.31 (3H, m, 2, 3-H), 4.86 (1H, t, J=1.7 Hz, 1-H)

The assignment numbers of the ¹H-NMR peaks are as follows. 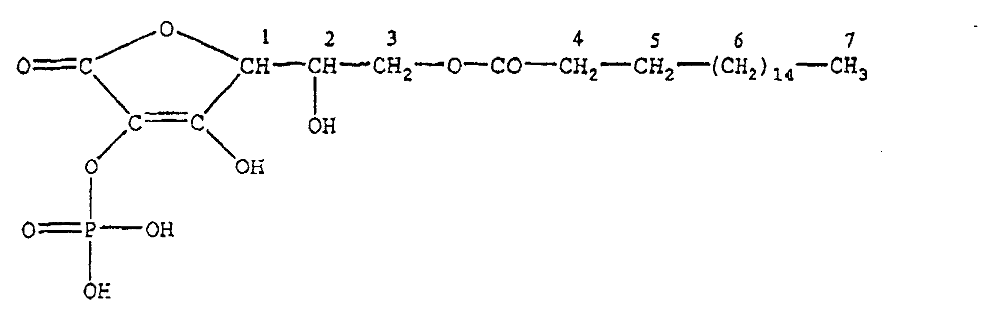 ¹³C - NMR (100 MHz, CD₃OD) δ: 14.4 (1C, s, 13-C), 23.7 (1C, s, 12-C), 26.0 (1C, s, 11-C), 30.2-30.7 (12C, s, 10-C), 33.0 (1C, s, 9-C), 34.8 (1C, s, 8-C), 65.6 (1C, s, 7 C), 68.0 (1C, s, 6-C), 77.3 (1C, s, 5-C), 115.3 (1C, d, J=6.1 Hz, 4-C), 160.3 (1C, d, J=3.8 Hz, 3-C), 170.5 (1C, d, J=6.1 Hz, 2-C), 175.1 (1C, s, 1-C)

The assignment numbers of the ¹³C-NMR peaks are as follows.

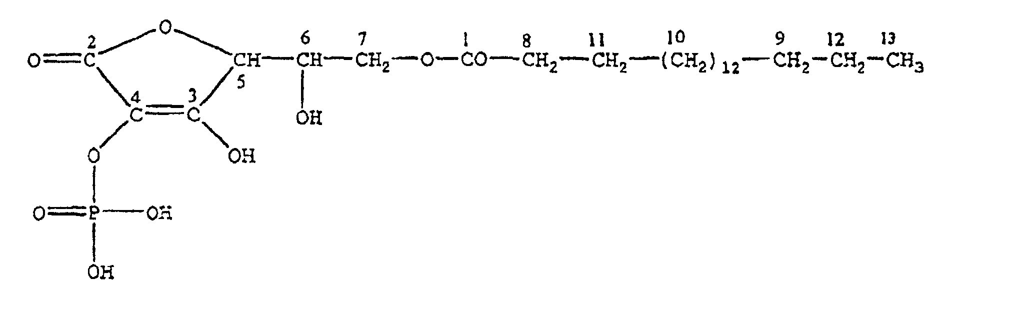

### Example 4

### L-Ascorbic acid-2-phosphate-6-laurate:

The reaction was performed in the same manner as in Example 1, except for using sodium laurate in place of palmitic acid. As a result, 2.2 g of L-ascorbic acid-2-phosphate-6-laurate was obtained (yield: 50%).

Various analytic data of the compound thus prepared are shown below,
MS m/z = 439 [M+H], 461 [M+Na], 483 [M+2Na], 505 [M+3Na]
¹H-NMR (400 MHz, CD₃OD) δ: 0.90 (3H, t, J=6.9 Hz, 7-H), 1.29 (16H, s, 6-H), 1.63 (2H, hep, J=7.3 Hz, 5-H), 2.38 (2H, t, J=7.3 Hz, 4-H), 4.11-4.30 (3H, m, 2, 3-H), 4.86 (1H, t, J=1.7 Hz, 1-H)

The assignment numbers of the ¹H-NMR peaks are as follows. 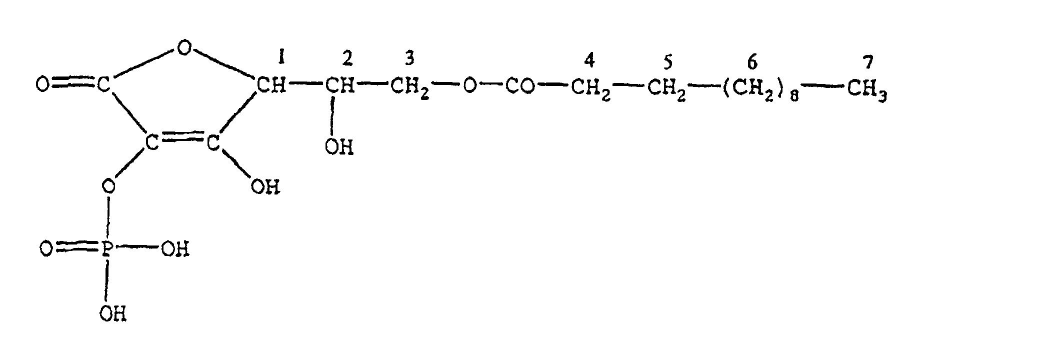 ¹³C-NMR (100 MHz, CD₃OD) δ: 14.4 (1C, s, 13-C), 23.7 (1C, s, 12-C), 25.9 (1C, s, 11-C), 30.1-30.7 (6C, s, 10-C), 33.0 (1C, s, 9-C), 34.8 (1C, s, 8-C), 65.5 (1C, s, 7-C), 67.9 (1C, s, 6-C), 77.2 (1C, s, 5-C), 115.2 (1C, d, J=5.4 Hz, 4-C), 160.5 (1C, d, J=3.8 Hz, 3-C), 170.5 (1C, d, J=6.1 Hz, 2-C), 175.1 (1C, s, 1-C)

The assignment numbers of the ¹³C-NMR peaks are as follows.

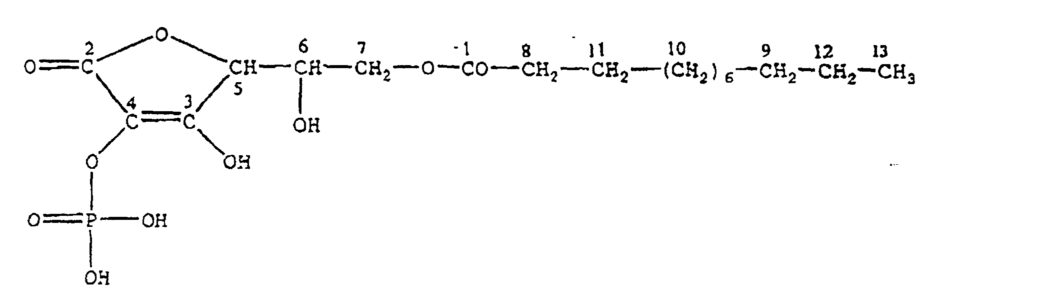

### Example 5

### L-Ascorbic acid-2-phosphate-6-palmitate magnesium salt:

The L-ascorbic acid-2-phosphate-6-palmitate of the present invention was added to purified water to a concentration of 2 mM, and magnesium oxide was gradually added thereto while stirring to effect neutralization (pH: about 8). As a result, L-ascorbic acid-2-phosphate-6-palmitate magnesium salt was obtained.

### Example 6

### L-Ascorbic acid-2-phosphate-6-palmitate sodium salt:

The L-ascorbic acid-2-phosphate-6-palimitate of the present invention was dissolved in methanol to a concentration of 20 mM, and the same volume of a 60 mM sodium hydroxide methanol solution was mixed therewith while stirring. The mixed solution was filtered and the precipitate was collected, washed with a small amount of methanol and dried under reduced pressure to obtain L-ascorbic acid-2-phosphate-6-palmitate sodium salt as a powder product.

## Claims

1. A process for producing an ascorbic acid derivative represented by the following formula (1) 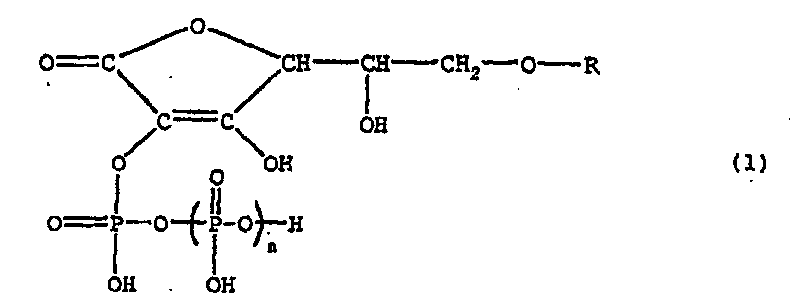 wherein R represents an acyl group having eleven or more carbon atoms and n is 0, 1 or 2 or a salt thereof, which comprises reacting an ascorbic acid-2-phosphoric acid ester or 2-pyrophosphoric acid ester or 2-triphosphoric acid ester and/or a salt thereof with at least one compound selected from fatty acids, fatty acid esters and salts thereof.

2. The process for producing an ascorbic acid derivative according to claim 1, which comprises reacting an ascorbic acid-2-phosphoric acid ester and/or a salt thereof with at least one of a fatty acid, an ester thereof and a salt thereof to produce a compound according to claim 1, wherein n is 0.

3. The process for producing an ascorbic acid derivative according to claim 1 or 2, wherein said reaction is performed in the presence of a condensing agent.

4. The process for producing an ascorbic acid derivative according to claim 1 or 2, wherein said reaction is performed in the presence of concentrated sulfuric acid.

## Patentansprüche

1. Verfahren zur Herstellung eines Ascorbinsäurederivats, das durch die folgende Formel (1) 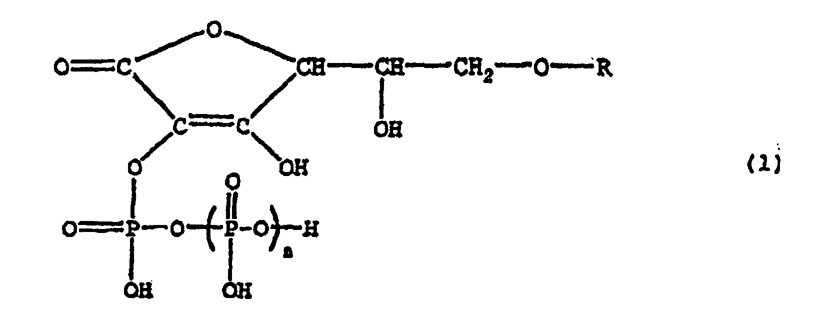 dargestellt ist, oder eines Salzes davon, welches die Umsetzung eines Ascorbinsäure-2-phosphorsäureesters oder 2-Pyrophosphorsäureesters oder 2-Triphosphorsäureesters und/oder eines Salzes davon mit mindestens einer Verbindung umfaßt, die unter Fettsäuren, Fettsäureestern und deren Salzen ausgewählt ist.

2. Verfahren zur Herstellung eines Ascorbinsäurederivats nach Anspruch 1, welches die Umsetzung eines Ascorbinsäure-2-phosphorsäureesters und/oder eines Salzes davon mit mindestens einer der folgenden Verbindungen: einer Fettsäure, eines Esters davon und eines Salzes davon, unter Bildung einer Verbindung nach Anspruch 1 umfaßt, worin n 0 ist.

3. Verfahren zur Herstellung eines Ascorbinsäurederivats nach Anspruch 1 oder 2, wobei die Reaktion in Gegenwart eines Kondensationsmittels durchgeführt wird.

4. Verfahren zur Herstellung eines Ascorbinsäurederivats nach Anspruch 1 oder 2, wobei die Reaktion in Gegenwart von konzentrierter Schwefelsäure durchgeführt wird.

## Revendications

1. Procédé pour la production d'un dérivé d'acide ascorbique représenté par la formule suivante (1) 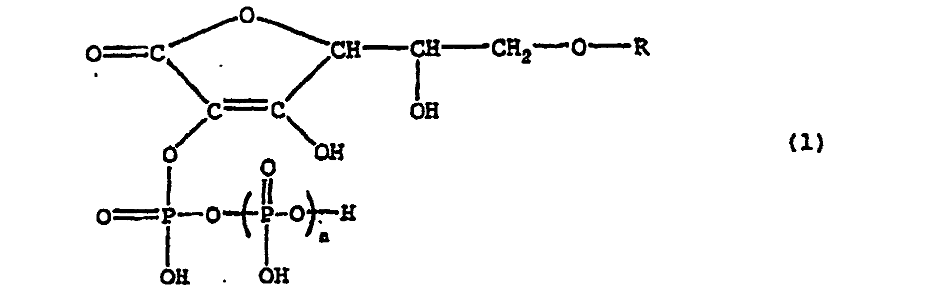 où R représente un groupe acyle ayant 11 atomes de carbone ou plus et n vaut 0, 1 ou 2 ou un sel de celui-ci, qui comprend la réaction d'un ester 2-phosphate ou d'un ester 2-pyrophosphate ou d'un ester 2-triphosphate de l'acide L-ascorbique et/ou d'un sel de ces esters avec au moins un composé choisi parmi des acides gras, des esters d'acides gras et des sels de ceux -ci.

2. Procédé pour la production d'un dérivé d'acide ascorbique selon la revendication 1, qui comprend la réaction d'un ester 2-phosphate de l'acide L-ascorbique et/ou d'un sel de celui-ci avec au moins un composé parmi un acide gras, un ester d'acide gras et un sel d'acide gras, pour produire un composé selon la revendication 1, où n vaut 0.

3. Procédé pour la production d'un dérivé d'acide ascorbique selon la revendication 1 ou 2, dans lequel ladite réaction est réalisée en présence d'un agent condensant.

4. Procédé pour la production d'un dérivé d'acide ascorbique selon la revendication 1 ou 2, dans lequel ladite réaction est réalisée en présence d'acide sulfurique concentré.
